# EUROPEAN PATENT SPECIFICATION

(11) **EP 2 550 955 B1**
(45) Date of publication and mention of the grant of the patent: **21.12.2016**
(21) Application number: 10848442.9
(22) Date of filing: 26.04.2010
(51) Int. Cl.: A61K 8/63, A61K 8/06, A61K 8/37, A61K 8/86, A61K 8/39, A61Q 19/08, A61K 8/891, A61K 8/92

(54) **EMULSION COMPOSITION**
EMULSIONSZUSAMMENSETZUNG
COMPOSITION POUR ÉMULSION

(30) Priority: 26.03.2010 JP 2010072089
(43) Date of publication of application: 30.01.2013
(73) Proprietor: Shiseido Company, Ltd., Chuo-ku Tokyo 104-8010 (JP)
(72) Inventor: FURUKAWARA, Tomomi, Yokohama-shi Kanagawa 224-8558 (JP); OMURA, Takayuki, Yokohama-shi Kanagawa 224-8558 (JP)
(74) Representative: Henkel, Breuer & Partner
(86) International application number: PCT/JP2010/057331
(87) International publication number: WO 2011/118050

(56) References cited:
- JP-A- 4 013 608
- JP-A- 2002 194 168
- JP-A- 2002 275 265
- JP-A- 2003 026 530
- JP-A- 2006 131 556
- JP-A- 2008 050 305
- JP-A- 2008 074 757
- JP-A- 2008 074 758
- JP-A- 2008 230 997

## Description

### Technical Field

The present invention relates to an emulsion composition. More particularly, the present invention relates to an emulsion composition having smooth texture, being able to confer resilient feel and emollient feel to the skin, as well as having superior stability of the formulation.

### Background Art

Objectives of skin care cosmetics are (1) to clean the skin, (2) to keep the moisture balance of the skin, (3) to activate the metabolism of the skin, (4) to protect the skin from harmful ultraviolet rays and the like. Skin care cosmetics are provided with functions that perform the above objectives to allow the natural function of the skin to work normally, as a result, maintain and recover healthy and beautiful skin, for example, various functions such as cleansing/cleaning, anti-dry, anti-ultraviolet rays, anti-oxidation, activation, as well as whitening, prevention of wrinkles/sags, acne prevention (Non Patent Document 1).

Patent Document 1 describes the emulsion cosmetic containing specific olefin polymers and a water-holding oil which is in the form of paste at 20°C, indicating that it has a superior adherence property, high moisturizing effect and its persistency, and provides soft films to give a moderate resilient feel.

Phytosterol derivatives part of which is included in the water-holding oil used in the emulsion cosmetic described above are described that when contained in an agent for external application on skin together with active ingredients such as vitamin A, vitamin E or their derivatives, they deliver those active ingredients to deep stratum corneum, and have an effect of conditioning the skin to prevent rough skin (Patent Document 2).

Bases containing silicone oils such as methyl polysiloxane are known to provide a smooth feeling during use. However, in the base containing methyl polysiloxane and exerting smooth texture, containing one kind of the above mentioned phytosterol derivatives, macadamia nut oil fatty acid phytosteryl, unfortunately causes a problem that crystals of macadamia nut oil fatty acid phytosteryl precipitate, leading to poor formulation stability though persistent effects of an resilient feel/emollient feel may be obtained.

Patent Document 3 discloses a solid powder cosmetic having improved ultraviolet light protecting potential, comprising 0.1 to 2% by mass of silylated silicic acid anhydride. In this context, a foundation comprising, among others, 2% by mass of macadamia nut oil fatty acid phytosteryl, 1% by mass of methyl polysiloxane, and 6% by mass of methylphenyl polysiloxane is described.

Patent Document 4 discloses a solid powder cosmetic providing natural and smooth finish, comprising a composite powder consisting of titanated mica coated with iron oxide and titanium dioxide. In this context, a foundation comprising, among others, 2% by mass of macadamia nut oil fatty acid phytosteryl, 1% by mass of methyl polysiloxane, and 6% by mass of methylphenyl polysiloxane is mentioned.

Patent Document 5 discloses a skin care preparation having anti-aging properties, comprising an extract of a plant of the genus Duabanga, and at least one of an oligopeptide and an acylated derivative thereof. Example 6 describes an essence comprising, among others, 1.5% by mass of macadamia nut oil fatty acid phytosteryl, 1% by mass of dimethyl polysiloxane, and 6% by mass of glyceryl tri(2-ethylhexanoate).

Patent Document 6 discloses a skin care preparation having anti-aging properties, comprising an extract of a plant of the genus Duabanga, and at least one of vitamin A and a derivative thereof. Example 7 mentions an essence comprising, among others, 1.5% by mass of macadamia nut oil fatty acid phytosteryl, 1% by mass of dimethyl polysiloxane, and 6% by mass of glyceryl tri(2-ethylhexanoate).

Patent Document 7 discloses an emulsion composition free from flocculation and oil separation, comprising a homopolymer or/and a copolymer of a sugar-derived monomer, a water-soluble polymer, an oil component, and water. Example 24 describes a lotion comprising, in addition to the aforementioned compounds, 3% by mass of macadamia nut oil fatty acid phytosteryl, 1% by mass of dimethyl polysiloxane, and 3% by mass of glyceryl tri(2-ethylhexanoate).

Patent Document 8 discloses an external cosmetic preparation for improving skin roughness and for protecting and restoring hair, comprising at least one polyacylated gluconic acid amid derivative. Example 19 mentions a lotion comprising, in addition to the aforementioned compound, 5% by mass of macadamia nut oil fatty acid phytosteryl, 2% by mass of dimethyl polysiloxane, and 5% by mass of glyceryl tri(2-ethylhexanoate).

### Prior Art Documents

### Patent Documents

Patent Document 1: JP-A 2008-247833
Patent Document 2: JP-A 2009-249342
Patent Document 3: JP-A 2006-131556
Patent Document 4: JP-A 2008-230997
Patent Document 5: JP-A 2008-074757
Patent Document 6: JP-A 2008-074758
Patent Document 7: JP-A 2002-194168
Patent Document 8: JP-A 2003-026530

### Non Patent Document

Non Patent Document 1: "Shin Kesyouhin Gaku (New Cosmetic Science)", 2nd ed., edited by Takeo Mitsui, NANZANDO Co., Ltd., 2001, p.345-347

### Summary of Invention

### Problem to be solved by the Invention

The present invention has been made in the light of the above situation, and aims to provide an emulsion composition that keeps smooth usability due to methyl polysiloxane, prevents the crystal precipitation of macadamia nut oil fatty acid phytosteryl to enhance formulation stability, and has a superior effect of conferring a resilient feel and emollient property.

### Means for solving the Problem

To solve such a problem, the present inventors, as a result of diligent research, have found that the crystal precipitation of macadamia nut oil fatty acid phytosteryl can be inhibited by admixing specific oils in a predetermined ratio to the macadamia nut oil fatty acid phytosteryl, thus completing the present invention.

The present invention thus provides an emulsion composition comprising:
(A) 0.05-3% by mass of macadamia nut oil fatty acid phytosteryl,
(B) 1-5% by mass of methyl polysiloxane, and
(C) one or more oils selected from the group consisting of glyceryl tri(2-ethylhexanoate), methylphenyl polysiloxane, cetyl ethylhexanoate, pentaerythrityl tetraethylhexanoate, and tripropylene glycol pivalate, wherein the content ratio of (A) and (C), (A):(C), is 1:3 to 1:200, and wherein the total content of oils in the emulsion composition is 1-8% by mass.

### Effects of Invention

According to the present invention, a stable emulsion composition can be obtained that while retaining smooth, fresh and refreshing feeling during use due to containing methyl polysiloxane, inhibits crystallization of macadamia nut oil fatty acid phytosteryl and is superior in resilient feel and emollient property.

### Modes for carrying out the Invention

The emulsion cosmetic of the present invention contains macadamia nut oil fatty acid phytosteryl (ingredient A) as an essential ingredient.

Macadamia nut oil fatty acid phytosteryl is an esterified product of macadamia nut oil and phytosterol, and is materials derived from plants which has a similar structure to intercellular lipids and can form a lamellar liquid crystal by itself.

The macadamia nut oil fatty acid phytosteryl in the present invention is not limited specifically, but may be those commercially available ones including, for example, YOFCO-MAS (from NIPPON FINE CHEMICAL CO.,LTD.).

The content of macadamia nut oil fatty acid phytosteryl (ingredient A) in the emulsion composition of the present invention is 0.05-3% by mass. The content of it less than 0.001% by mass causes difficulty in conferring a sufficient resilient feel and emollient property to skin, while the content of it greater than 10% by mass may cause poor stability of the formulation.

The methyl polysiloxane (ingredient B) contained in the emulsion composition of the present invention as an essential ingredient is not limited specifically, but preferably those having relatively low viscosity (for example, viscosity at 25°C equal to or less than 100 cs) are used.

The methyl polysiloxane used in the present invention may be those commercially available, for example, Silicone KF-96A-100cs, Silicone KF-96A-10cs, Silicone KF-96A-20cs, Silicone KF-96A-30cs, Silicone KF-96A-50cs, Silicone KF-96A-5cs, Silicone KF-96A-6cs, Silicone KF-96A-1.5cs, and Silicone KF-96A-2cs (all of which are from Shin-Etsu Chemical Co., Ltd.).

The content of a methyl polysiloxane (ingredient B) in the emulsion composition of the present invention is 1-5% by mass. The content of it less than 0.1% by mass causes insufficient smoothness and freshness, while the content of it greater than 30% by mass may cause poor stability of the formulation.

The emulsion composition of the present invention contains, as essential ingredients, one or more oils (ingredient C) selected from glyceryl tri(2-ethylhexanoate), methylphenyl polysiloxane, cetyl ethylhexanoate, pentaerythrityl tetraethylhexanoate, and tripropylene glycol pivalate.

The above described oils are preferably selected from, the oils listed above, but may be replaced by other oils as long as they have the equivalent characteristics and successfully achieve the advantage of the present invention.

The content of oils (ingredient C) described above in the emulsion composition of the present invention will be adjusted such that the content ratio of macadamia nut oil fatty acid phytosteryl (ingredient A) to the ingredient C, (A): (C), is 1:3 to 1:200, preferably 1:3 to 1:150, and more preferably 1:3 to 1:100.

The content of the ingredient C less than 3 times the content of macadamia nut oil fatty acid phytosteryl cannot effectively inhibit the crystal precipitation of macadamia nut oil fatty acid phytosteryl. On the contrary, the content of it greater than 200 times the content of macadamia nut oil fatty acid phytosteryl does not further improve the characteristics of the formulation.

The emulsion composition of the present invention preferably further contains (D) a polyethylene glycol (PEG) in addition to the above essential ingredients A to C. Containing a PEG further improves an emollient property.

The PEG used in the present invention is not limited specifically, but preferably used are, for example, PEG-75, PEG-150, PEG-6, PEG-20, and PEG-400.

When contained in the emulsion composition of the present invention, the content of a PEG is typically 0.01-30% by mass, and preferably 0.5-15% by mass.

The emulsion composition of the present invention may contain, as appropriate, agents for external application on skin and other ingredients that may be typically contained in cosmetics, other than the essential ingredients described above and the PEG, without disrupting the advantages of the present invention.

Other ingredients may include, but not especially limited to, alcohols, thickeners, neutralizers, liquid oils (except the ingredients B and C described above), solid oils, semisolid oils, surfactants, chelating agents, preservatives, flavors, ultraviolet absorbing agents, medicaments, moisturizing agents, powders and the like.

In the emulsion composition of the present invention, the total content of oils contained in the formulation (including the ingredients B and C described above) is 1-8% by mass. The total content of oils less than 1% does not provide sufficient smoothness and an emollient property, while the total content of oils greater than 30% may cause poor stability of the base.

The emulsion composition of the present invention may be in either form of oil-in-water or water-in-oil, which can be selected as appropriate depending on applications and purposes, and prepared according to routine methods used for a composition in such form.

The emulsion composition of the present invention may be provided as an emulsion cosmetic in the form of a lotion, a milky lotion, a cream, a gel, and a beauty lotion (essence) and the like.

### Examples

The present invention will be described in more detail below with reference to specific examples, but the present invention is not intended to be limited to these examples. It is noted that in the following examples contents are expressed in % by mass, unless otherwise stated.

### (Examples and Comparative examples)

Samples with the compositions listed in tables 1 and 2 below were prepared, and evaluated for their texture (smoothness and resilient feel) when used and the crystal precipitation of macadamia nut oil fatty acid phytosteryl.

For their texture, each sample was evaluated by 15 special panelists by using it, according to the following criteria:
○: Not less than 10 panelists answered that the sample has smoothness/resilient feel.
Δ: 5-9 panelists answered that the sample has smoothness/resilient feel.
×: Less than 4 panelists answered that the sample has smoothness/resilient feel.

For the crystal precipitation of macadamia nut oil fatty acid phytosteryl, the presence or absence of the crystal precipitation was determined by observation with microscope of each sample disposed between polarizing plates.
○: The crystal precipitation was not observed.
×: The crystal precipitation was observed.

**[Table 1]**

| classification | material names | Comparative example 1 | Comparative example 2 | Example 1 | Example 2 |
|---|---|---|---|---|---|
| | purified water | balance | balance | balance | balance |
| | ethanol | 8 | 8 | 8 | 8 |
| | glycerin | 5 | 5 | 5 | 5 |
| | PEG-75 | 7 | 7 | 7 | 7 |
| | methyl gluceth-10 | 2 | 2 | 2 | 2 |
| | maltitol | 1 | 1 | 1 | 1 |
| | carboxyvinyl polymer | 0.15 | 0.15 | 0.15 | 0.15 |
| | acrylic acid/alkyl acrylate (C10-30) copolymer | 0.05 | 0.05 | 0.05 | 0.05 |
| | potassium hydroxide | 0.07 | 0.07 | 0.07 | 0.07 |
| (B) | methyl polysiloxane | 3 | 3 | 3 | 3 |
| | glyceryl tri(2-ethylhexanoate) | 0.5 | 0.5 | 1 | 0.5 |
| (C) | methylphenyl polysiloxane | - | - | - | 0.5 |
| | tripropylene glycol pivalate | - | - | - | - |
| (A) | macadamia nut oil fatty acid phytosteryl | - | 0.2 | 0.2 | 0.2 |
| | EDTA-2Na.2H2O | 0.03 | 0.03 | 0.03 | 0.03 |
| | phenoxyethanol | 0.5 | 0.5 | 0.5 | 0.5 |
| | perfume | q.s. | q.s. | q.s. | q.s. |
| | | | | | |
| content ratio | (A):(C) | - | 1:2.5 | 1:5 | 1:5 |
| | | | | | |
| texture | smoothness | ○ | ○ | ○ | ○ |
| | resilient feel | × | ○ | ○ | ○ |
| | | | | | |
| crystal precipitation of macadamia nut oil fatty acid phytosteryl | | - | × | ○ | ○ |

**[Table 2]**

| classification | material names | Example 3 | Example 4 | Example 5 | Comparative example 3 |
|---|---|---|---|---|---|
| | purified water | balance | balance | balance | balance |
| | ethanol | 8 | 8 | 8 | 8 |
| | glycerin | 5 | 5 | 5 | 5 |
| | PEG-75 | 7 | 7 | 7 | 7 |
| | methyl gluceth-10 | 2 | 2 | 2 | 2 |
| | maltitol | 1 | 1 | 1 | 1 |
| | carboxyvinyl polymer | 0.15 | 0.15 | 0.15 | 0.15 |
| | acrylic acid/alkyl acrylate (C10-30) copolymer | 0.05 | 0.05 | 0.05 | 0.05 |
| | potassium hydroxide | 0.07 | 0.07 | 0.07 | 0.07 |
| (B) | methyl polysiloxane | 3 | 3 | 3 | - |
| | glyceryl tri(2-ethylhexanoate) | 0.5 | 5 | 1 | 0.5 |
| (C) | methylphenyl polysiloxane | - | - | 4 | 2.5 |
| | tripropylene glycol pivalate | 0.5 | - | - | - |
| (A) | macadamia nut oil fatty acid phytosteryl | 0.1 | 0.05 | 1 | 0.2 |
| | EDTA-2Na.2H2O | 0.03 | 0.03 | 0.03 | 0.03 |
| | phenoxyethanol | 0.5 | 0.5 | 0.5 | 0.5 |
| | perfume | q.s. | q.s. | q.s. | q.s. |
| | | | | | |
| content ratio | (A):(C) | 1:10 | 1:100 | 1:5 | 1:15 |
| | | | | | |
| texture | smoothness | ○ | ○ | ○ | × |
| | resilient feel | ○ | ○ | ○ | ○ |
| | | | | | |
| crystal precipitation of macadamia nut oil fatty acid phytosteryl | | ○ | ○ | ○ | ○ |

As is apparent from the results shown in the tables 1 and 2, an resilient feel was not obtained in the comparative example 1 which did not contain macadamia nut oil fatty acid phytosteryl (A), and the comparative example 2 in which the content ratio of a macadamia nut oil fatty acid phytosteryl (A) and oils (C) is not in the specified range did not inhibit the crystal precipitation of macadamia nut oil fatty acid phytosteryl. Also, a smooth feeling during use was not obtained with the comparative example 3 which does not contain methyl polysiloxane (B). On the other hand, a smooth feeling during use and resilient feel were obtained with the emulsion compositions of the present invention (Examples 1-5), and these compositions were stable as crystal precipitation of macadamia nut oil fatty acid phytosteryl was not observed.

Formulation examples of the cosmetics comprising the emulsion composition of the present invention are listed below. All of these cosmetics were stable cosmetics that provided smooth, fresh and refreshing feeling during use, a resilient feel and superior emollient effect, and did not cause crystal precipitation.

### Example 6: beauty lotion

| Purified water | balance |
|---|---|
| Ethanol | 10 |
| Glycerin | 5 |
| Butylene glycol | 1 |
| PEG-6 | 2 |
| Maltitol | 1 |
| Carboxylvinyl polymer | 0.2 |
| Acrylic acid/alkyl acrylate (C10-30) copolymer | 0.05 |
| Potassium hydroxide | 0.1 |
| Methyl polysiloxane | 3 |
| Glyceryl tri (2-ethylhexanoate) | 0.5 |
| Methylphenyl polysiloxane | 0.5 |
| Macadamia nut oil fatty acid phytosteryl | 0.1 |
| Potassium 4-methyl salicylate | 1 |
| Vitamin E derivative | 0.05 |
| EDTA-2Na | 0.03 |
| Paraben | 0.2 |
| Flavor | q.s. |

### Method of manufacture:

Oil-soluble ingredients were dissolved in oil warmed to 60°C (oil phase). On the other hand, water-soluble ingredients were dissolved in purified water (aqueous phase). The above oil phase was added to the aqueous phase, and then mixed by stirring.

### Example 7: gel cream

| Purified water | balance |
|---|---|
| Ethyl alcohol | 8 |
| Glycerin | 8 |
| PEG-150 | 3 |
| Carboxyvinyl polymer | 0.45 |
| Acrylic acid/alkyl (C10-30) acrylate copolymer | 0.1 |
| Aminomethyl propanediol | 0.1 |
| Methyl polysiloxane | 4 |
| Cetyl ethylhexanoate | 1 |
| Pentaerythrityl tetraethylhexanoate | 1 |
| Macadamia nut oil fatty acid phytosteryl | 0.5 |
| Tranexamic acid | 1 |
| Dipotassium glycyrrhizinate | 0.05 |
| Phenoxyethanol | 0.5 |
| Flavor | q.s. |

### Method of manufacture:

Oil-soluble ingredients were dissolved in oil, and then this oil was warmed to 60°C (oil phase). On the other hand, water-soluble ingredients were dissolved in purified water (aqueous phase). The above oil phase was added to the aqueous phase, and the combined was mixed by stirring to obtain the gel cream.

### Example 8: a milky lotion

| Purified water | balance |
|---|---|
| Ethyl alcohol | 5 |
| Glycerin | 5 |
| Butylene glycol | 0.5 |
| Maltitol | 0.5 |
| PEG-75 | 7 |
| Carboxyvinyl polymer | 0.15 |
| Acrylic acid/alkyl (C10-30) acrylate copolymer | 0.1 |
| Potassium hydroxide | 0.1 |
| Polyoxyethylene/methyl polysiloxane copolymer | 0.1 |
| Methyl polysiloxane | 3 |
| Pentaerythrityl tetraethylhexanoate | 0.5 |
| Tripropylene glycol pivalate | 1 |
| Macadamia nut oil fatty acid phytosteryl | 0.1 |
| EDTA-2Na | 0.03 |
| Phenoxyethanol | 0.5 |
| Flavor | q.s. |

### Method of manufacture:

Oil-soluble ingredients were dissolved in oil warmed to 60°C (oil phase). On the other hand, water-soluble ingredients were dissolved in purified water (aqueous phase). The above oil phase was added to the aqueous phase, and the combined was mixed by stirring.

## Claims

1. An emulsion composition comprising
(A) 0.05-3% by mass of macadamia nut oil fatty acid phytosteryl,
(B) 1-5% by mass of methyl polysiloxane, and
(C) one or more oils selected from the group consisting of glyceryl tri(2-ethylhexanoate), methylphenyl polysiloxane, cetyl ethylhexanoate, pentaerythrityl tetraethylhexanoate, and tripropylene glycol pivalate,
wherein the content ratio of (A) to (C), (A):(C), is 1:3 to 1:200, and wherein the total content of oils in the emulsion composition is 1-8% by mass.

2. The emulsion composition according to claim 1, further containing (D) polyethylene glycol.

3. An emulsion cosmetic comprising the emulsion composition according to claim 1 or 2.

## Patentansprüche

1. Emulsionszusammensetzung, umfassend:
(A) 0.05-3 Masse% an Macadamianussölfettsäure-Phytosterolester,
(B) 1-5 Masse% an Methylpolysiloxan, und
(C) ein oder mehrere Öle ausgewählt aus der Gruppe bestehend aus Glyceryl-tri(2-ethylhexanoat), Methylphenylpolysiloxan, Cetylethylhexanoat, Pentaerythrityltetraethylhexanoat und Tripropylenglykolpivalat,
wobei das Anteilsverhältnis von (A) zu (C), (A):(C), 1:3 bis 1:200 beträgt, und wobei der Gesamtanteil an Ölen in der Emulsionszusammensetzung 1-8 Masse% beträgt.

2. Emulsionszusammensetzung nach Anspruch 1, weiterhin umfassend (D) Polyethylenglykol.

3. Emulsionskosmetikum, umfassend die Emulsionszusammensetzung nach Anspruch 1 oder 2.

## Revendications

1. Composition pour émulsion comprenant
(A) 0,05 à 3 % en masse de l'ester phytostéryle d'acide gras d'huile de noix de macadamia,
(B) 1 à 5 % en masse de polysiloxane de méthyle, et
(C) une ou plusieurs huiles choisies dans le groupe constitué par le tri(2-éthylhexanoate) de glycéryle, le polysiloxane de méthylphényle, l'éthylhexanoate de cétyle, le tétraéthylhexanoate de pentaérythrityle et le pivalate de tripropylène glycol,
dans laquelle le rapport des teneurs entre (A) et (C), (A) : (C), est de 1 : 3 à 1 : 200, et dans laquelle la teneur totale en huiles dans la composition pour émulsion est de 1 à 8 % en masse.

2. Composition pour émulsion selon la revendication 1, contenant en outre (D) du polyéthylène glycol.

3. Produit cosmétique en émulsion comprenant la composition pour émulsion selon la revendication 1 ou 2.
